# EUROPÄISCHE PATENTSCHRIFT

(11) **EP 1 646 408 B1**
(45) Veröffentlichungstag und Bekanntmachung des Hinweises auf die Patenterteilung: **27.12.2006**
(21) Anmeldenummer: 04740589.9
(22) Anmeldetag: 02.07.2004
(51) Int. Cl.: A61L 9/20, B01J 35/04, B01D 53/86, B01J 35/00

(54) **VORRICHTUNG ZUR REINIGUNG SCHADSTOFFHALTIGER ABLUFT**
DEVICE FOR PURIFYING USED AIR CONTAINING HARMFUL SUBSTANCES
DISPOSITIF POUR NETTOYER DE L'AIR D'EVACUATION CONTENANT DES CONTAMINANTS

(30) Priorität: 03.07.2003 DE 10330114
(43) Veröffentlichungstag der Anmeldung: 19.04.2006
(73) Patentinhaber: Schröder, Werner, 31542 Bad Nenndorf (DE)
(72) Erfinder: Schröder, Werner, 31542 Bad Nenndorf (DE)
(74) Vertreter: Joppich, Martin
(86) Internationale Anmeldenummer: PCT/EP2004/007237
(87) Internationale Veröffentlichungsnummer: WO 2005/002638

(56) Entgegenhaltungen:
- EP-A- 0 325 133
- EP-A- 0 778 070
- CA-A- 2 249 924
- FR-A- 2 308 409
- GB-A- 2 367 495
- US-A- 2 071 119
- US-A- 2 413 704
- US-A- 5 933 702
- US-A1- 2002 160 913
- US-A1- 2003 086 831

## Beschreibung

Die Erfindung betrifft eine Vorrichtung zur Reinigung schadstoffhaltiger Abluft in einem Abluftkanal.

Eine derartige Vorrichtung zur Reinigung schadstoffhaltiger Abluft ist aus EP 0 778 080 B1 bekannt.

Darüber hinaus betrifft die Erfindung eine Reaktionsstufe eines Abluftkanals mit mehreren Luftleitkanälen, in denen längs zur Luftströmung der Abluft jeweils ein röhrenförmiger UV-Strahler angeordnet ist.

Eine derartige Reaktionsstufe eines Abluftkanals ist aus JP 07-060058 A bekannt.

Aus EP 0 778 080 B1 ist es bekannt, Schadstoffe wie Lösungsmittel oder Geruchsstoffe in einer Reaktionsstufe photooxidativ durch Bestrahlung der Abluft mit energiereichem UVC-Licht in einem Luftleitkanal umzusetzen. Hierbei ist es grundsätzlich auch bekannt, zur Steigerung der Effektivität mehrere Luftleitkanäle parallel zu schalten. Infolge der Wechselwirkung von UVC-Strahlung und Abluft entstehen die für den Schadstoffabbau benötigten reaktiven Spezies. Durch die Absorption von UVC-Licht durch Sauerstoff- und Wassermoleküle der Abluft entstehen die Oxidationsmittel Ozon, Wasserstoffperoxid sowie O- und OH-Radikale. Diese besitzen hohe Oxidationspotentiale und sind daher in der Lage, Schadstoffe zu oxidieren. Hierbei wird eine Kettenreaktion angestoßen, bei der neue Radikale entstehen, die ihrerseits wieder andere Moleküle angreifen können. Daneben erfolgt eine Absorption der UVC-Strahlung durch die Schadstoffmoleküle und deren Zerfallsprodukte. Durch die Absorption der Lichtenergie werden die Schadstoffe auf höhere energetische Niveaus angeregt und damit für eine Umsetzung mit den reaktiven Spezies oder auch mit Luftsauerstoff aktiviert. Bei genügend hoher Zufuhr von Lichtenergie kommt es zum Zerfall des Moleküls. Die Zerfallsprodukte der Schadstoffphotolyse können ebenfalls OH-Radikale bilden oder radikalische Kettenreaktionen anstoßen. Infolge der Lichtanregung und der Gegenwart reaktiver Sauerstoffverbindungen starten homogene Gasphasenreaktionen.

In Kombination zu dieser photooxidativen Umsetzung befindet sich im Anschluss an die Reaktionsstufe eine Katalysatoreinheit, welche zusätzliche Abbaureaktionen erlaubt und in welcher überschüssiges Ozon abgebaut wird, so dass sichergestellt ist, dass das Schadgas Ozon nicht in die Umwelt gelangt.

Der aus der EP 0 778 070 B1 bekannte Katalysator ist vorzugsweise ein Aktivkohle-Katalysator. Die zur Anwendung kommende Aktivkohle ist ein hochporöses Material mit einer inneren Oberfläche von ca. 1200 m²/g, die als Reaktionsoberfläche genutzt werden. Die Aufgabe der Aktivkohle besteht zum einen darin, schwer oxidierbare Verbindungen zurückzuhalten und damit ihre Verweilzeit im Reaktor zu erhöhen. Dadurch wird die Konzentration dieser Komponenten im Vergleich zur Gasphase erhöht, was zu einer Steigerung der Reaktionsgeschwindigkeit mit den gebildeten Sauerstoffspezies auf der Aktivkohle-Oberfläche führt. Zum anderen wird durch die Verwendung der Aktivkohle als nachgeschalteter Katalysator sichergestellt, dass das Schadgas Ozon nicht in die Umwelt gelangt, da Aktivkohle als Ozonfilter wirkt.

Zur Erzeugung der UV-Strahlung gemäß der EP 0 778 070 B1 werden üblicherweise röhrenförmige UV-Strahler verwendet. Die EP 0 778 070 B1 lässt es dabei offen, wie die UV-Strahler in der photooxidativen Reaktionsstufe angeordnet sein können. Aus dem Stand der Technik sind allerdings auch bereits entsprechende Reaktionsstufen bekannt, die bevorzugte Anordnungen der UV-Strahler vorschlagen.

Aus JP 07-060-058 A ist eine Vorrichtung zur Reinigung schadstoffhaltiger Abluft in einem Abluftkanal bekannt, bei der ein UV-Strahler in einem Luftleitkanal parallel zur Luftströmung angeordnet ist und dessen UV-Strahlung Wellenlängen sowohl im Bereich von 185 nm als auch im Bereich von 254 nm aufweist. Zusätzlich schlägt die JP 07-060058 A vor, die Innenwände des Luftleitkanals mit Titandioxyd zu beschichten, um in der gleichen Reaktionsstufe bereits eine Katalysatorwirkung zu erzielen.

Aus DE 197 40 053 A1 ist eine weitere Vorrichtung zur Reinigung schadstoffhaltiger Abluft in einem Abluftkanal bekannt, bei der mehrere röhrenförmige UV-Strahler in der photooxdativen Reaktionsstufe ebenfalls parallel zum Luftstrom angeordnet sind. Die DE 197 40 053 A1 erwähnt ebenfalls die zusätzliche Verwendung von Titandioxyd als Katalysator und schlägt für eine ausreichende Wechselwirkung zwischen den in der Abluft enthaltenen Schadstoffen und der UV-Strahlung entsprechende Umlenk-und/oder Lochbleche vor.

Aus US 2002/0160913 A1 ist eine Reaktionsstufe eines Abluftkanals bekannt, die mehrere parallel geschaltete Luftleitkanäle mit einem quadratischen Querschnitt aufweist. Die Oberfläche der Luftleitkanäle kann mit einem Photokatalysator beschichtet sein. Ein UV-Strahler kann vor den Luftleitkanälen, aber nicht innerhalb der Luftleitkanäle angeordnet sein.

Aus US 2,413,704 ist eine Reaktionsstufe eines Abluftkanals bekannt, der aus einem Luftleitkanal mit einem runden Querschnitt besteht, in dem mehrere röhrenförmige UV-Strahler längs zur Luftströmung angeordnet sind.

Es hat sich gezeigt, dass die Verfügbarkeit einer kostengünstigen, kompakten Abluftreinigungsanlage insbesondere für kleine Produktionseinheiten immer mehr an Bedeutung gewinnt. Ausgehend von der aus der JP 07-060058 bekannten Vorrichtung ist es daher Aufgabe der Erfindung, die Abbaurate auf einfache Weise zu steigern, mit der die schadstoffbelastete Abluft innerhalb des Luftleitkanals gereinigt und von Schadstoffen befreit wird, um somit eine kostengünstige und kompakte Abluftreinigungsanlage zur Verfügung stellen zu können.

Diese Aufgabe wird durch eine Reaktionsstufe eines Abluftkanals gemäß dem Patentanspruch 1 und eine Vorrichtung zur Reinigung schadstoffhaltiger Abluft gemäß dem Patentanspruch 15 gelöst.

Eine wesentliche Erkenntnis der Erfindung besteht darin, dass bei einer geeigneten Änderung der Geometrie des Querschnitts des aus der JP 07-060058 A bekannten Luftleitkanals eine bessere Wechselwirkung zwischen der UV-Strahlung, den in der Abluft enthaltenen Schadstoffen sowie dem auf den Innenwänden des Luftleitkanals beschichteten Katalysators erreicht werden kann. Die JP 07-060058 A schlägt einen quadratischen oder rechteckigen Querschnitt des Luftleitkanals vor. Im Gegensatz dazu hat die Erfindung gezeigt, dass eine Steigerung der Abbaurate innerhalb eines Luftleitkanals möglich ist, wenn der Querschnitt des mindestens einen Luftleitkanals ein regelmäßiges Vieleck mit mindestens 5 Seiten ist. Darüber hinaus ist erfindungsgemäß vorgesehen, dass mehrere Luftleitkanäle wabenförmig nebeneinander angeordnet sind. Durch mehrere Luftleitkanäle wird die Wirkung der Reaktionsstufe gesteigert, wobei die erfindungsgemäße Reaktionsstufe durch die wabenförmige Anordnung kompakt gebaut werden kann.

Zur Ausbildung der wabenförmigen Struktur bietet es sich an, dass der Querschnitt der Luftleitkanäle jeweils ein regelmäßiges Sechseck oder ein regelmäßiges Achteck ist.

Den Grenzfall der Erfindung bildet ein Querschnitt, bei dem das regelmäßige Vieleck als Kreis ausgebildet ist und damit quasi aus unendlich vielen Seiten besteht. Aus der Sicht der Effektivitätssteigerung ist dieser Grenzfall des kreisförmigen Querschnitts optimal, allerdings bleibt der Zwischenraum zwischen verschiedenen Luftleitkanälen ungenutzt, wenn mehrere Luftleitkanäle parallel geschaltet sein sollen. Als ein günstiger Kompromiss zwischen dem aus dem Stand der Technik bekannten rechteckigen Querschnitt und dem kreisförmigen Querschnitt hat sich daher zur Parallelschaltung mehrerer Luftleitkanäle die wabenförmige Struktur mit sechs- oder achteckigen Querschnitten herausgestellt.

Nach einer bevorzugten Ausführungsform ist vorgesehen, dass ein UV-Strahler in einem Luftleitkanal durch seitlich angebrachte Kontaktschienen gehalten wird. Die Kontaktschienen sind vorzugsweise derart ausgestaltet, dass eine einfache Wartung und Auswechselung der röhrenförmigen UV-Strahler möglich ist.

Nach einer weiteren bevorzugten Ausführungsform ist vorgesehen, dass die von einem UV-Strahler abgegebene Strahlung die Bildung von reaktiven Reaktionsmitteln wie Ozon und/oder sauerstoffhaltige Radikale in der entlangströmenden Abluft bewirkt. Es ist bekannt, dass eine derartige Wirkung insbesondere dann erzielt werden kann, wenn die Wellenlänge der von dem jeweiligen UV-Strahler abgegebenen Strahlung im Bereich von 185 nm liegt.

Nach einer weiteren bevorzugten Ausführungsform ist vorgesehen, dass die von einem UV-Strahler abgegebene Strahlung die Anregung der in der Abluft enthaltenen Kohlenwasserstoffe auf höhere energetische Niveaus bewirkt. Es ist bekannt, dass eine derartige Wirkung insbesondere dann erzielt werden kann, wenn die Wellenlänge der von dem jeweiligen UV-Strahler abgegebenen Strahlung im Bereich von 254 nm liegt.

Besonders vorteilhaft ist es damit, UV-Strahler zu verwenden, deren abgegebene Wellenlänge im Bereich der Absorptionsspektren von den in der Abluft enthaltenen gasförmigen Moleküle liegt, wobei sich hier die Verwendung der Wellenlängenbereiche von 185 nm und 254 nm anbietet, da diese Wellenlängenbereiche bereits mit den-herkömmlichen Quecksilberdampflampen zur Verfügung stehen. Um gleichzeitig die Bauform der Reaktionsstufe weiter zu verringern, kann zusätzlich eine Leistungserhöhung der jeweils verwendeten UV-Strahler vorgesehen sein. Dabei muss die Lichtintensität des leistungsstärkeren UV-Strahlers als Funktion der Wellenlänge bestimmt werden, damit weiterhin eine hinreichende Überlappung der Absorptionsspektren der Schadstoffmoleküle mit dem Emissionsspektrum der Lichtquelle vorliegt.

Eine weitere Erkenntnis der Erfindung besteht darin, die von dem UV-Strahler abgegebenen Wellenlängen nicht auf die Absorptionsspektren der in der Abluft enthaltenen gasförmigen Moleküle zu optimieren, sondern die Wellenlängen im Hinblick auf dasjenige Katalysatormaterial zu optimieren, das zur Beschichtung der Innenwände des Luftleitkanals verwendet werden kann. Ausgehend von der JP 07-060058 A betrifft diese Erkenntnis der Erfindung damit eine Reaktionsstufe eines Abluftkanals mit mindestens einem Luftleitkanal, in dem längs zur Luftströmung der Abluft ein röhrenförmiger UV-Strahler angeordnet ist und dessen Innenwände mit einem breitbandigen Halbleitermaterial als Katalysatormaterial beschichtet sind. In der JP 07-060058 A wird dabei als Katalysatormaterial Titandioxyd (TiO₂) verwendet.

Ausgehend von der aus der JP 07-060058 A bekannten Vorrichtung kann die vorliegende Aufgabe der Erfindung, die Abbaurate auf einfache Weise zu steigern, mit der die schadstoffbelastete Abluft innerhalb des Luftleitkanals gereinigt und von Schadstoffen befreit wird, auf der Grundlage der Beschichtung der Innenwände des Luftleitkanals mit einem Halbleitermaterial dadurch gelöst werden, dass die von dem jeweiligen UV-Strahler abgegebene Strahlung Wellenlängen aufweist, die größer als 254 nm- sind und deren abgegebene Strahlungsenergie im Wesentlichen größer oder gleich der Energiedifferenz zwischen Valenz-und Leitungsband des Halbleitermaterials ist.

Grundsätzlich führt die Bestrahlung eines Photohalbleiters mit Photonen, deren Energie größer oder gleich der Energiedifferenz zwischen Valenz- und Leitungsband des Halbleiters ist, zur Generierung von Elektronen/Loch-Paaren. Die entscheidende Erkenntnis der Erfindung liegt dabei darin, dass die von dem UV-Strahler abgegebenen Wellenlängen in der Nähe der Absorptionskante des Halbleiters für den Umsatz der photokatalytischen Reaktionen besonders wirkungsvoll sind und zu photokatalytischen Reaktionen führen. Maßgeblich sind damit nicht die Wellenlängenbereiche von 185 nm und 254 nm herkömmlicher Quecksilberdampflampen, sondern alternativ oder ergänzend Wellenlängenbereiche mit darüber liegenden Wellenlängen, deren abgegebene Strahlungsenergie dementsprechend geringer ist, die aber gleichzeitig noch dazu ausreicht, um die Energiedifferenz zwischen Valenz-und Leitungsband des Halbleitermaterials zu überwinden.

Für diese Photokatalyse sind prinzipiell alle Halbleiter mit Bandlücken etwa zwischen 2 eV und 4 eV geeignet, wie beispielsweise Titandioxyd (TiO₂), Zinkoxid (ZnO), Cadmiumsulfat (CdS), Zirkoniumdioxid (ZrO₂), Wolframtrioxid (WO₃), Cerdioxid (CeO₂), Strontiumtitantrioxid (SrTiO₃) oder Zirkoniumtitanoxid (ZrTiO₄). Besonders geeignet hat sich Titandioxyd (TiO₂) oder auch dotiertes Titandioxyd herausgestellt, da sich hier die Eigenschaften von Reaktivität, Umweltverträglichkeit, Langzeitstabilität und auch Kosteneffektivität gut vereinen lassen. Sämtliche Photohalbleiter lassen sich durch energieäquivalentes Licht der-Wellenlängen zwischen 340 nm und 500 nm aktivieren.

Allgemein hat sich gezeigt, dass die gewünschte Katalysatorwirkung im Bereich der erfindungsgemäßen Reaktionsstufe erzielt werden kann, wenn die Innenwände des Luftleitkanals mit einem breitbandigen Halbleitermaterial als Katalysatormaterial beschichtet sind. Für den jeweiligen UV-Strahler muss dabei gewährleistet sein, dass der Bereich der Wellenlänge der von dem UV-Strahler abgegebenen Strahlung derart gewählt wird, dass die abgegebene Strahlungsenergie zumindest größer oder gleich der Energiedifferenz zwischen Valenz- und Leitungsband des Halbleitermaterials ist.

Nach einer bevorzugten Ausführungsform besteht das Halbleitermaterial dabei in bekannter Weise aus Titandioxyd. Das Halbleitermaterial kann aber auch aus dotiertem Titandioxyd bestehen. Durch Bestrahlung des Titandioxyds bzw. dotierten Titandioxid mit UV-Strahlung, deren Energie größer oder gleich der Energiedifferenz zwischen Valenz- und Leitungsband des Halbleiters ist, werden zunächst Elektron/Loch-Paare im Halbleitermaterial generiert. Daraufhin kommt es zur Bildung sauerstoffhaltiger Radikale, die den Prozess der Oxidation von Schadstoffen wirkungsvoll unterstützen. Hierbei hat sich gezeigt, dass zur Erzielung einer optimalen Wechselwirkung zwischen der UV-Strahlung und dem Katalysatormaterial der Abstand zwischen dem UV-Strahler und den Innenwänden des Luftleitkanals zu beachten ist. Zur Optimierung eines erfindungsgemäßen Luftleitkanals wird also der Abstand immer so gewählt sein, dass sich bei gegebenem Katalysatormaterial und vorgegebenem UV-Strahler eine optimale Abbaurate der jeweiligen Schadstoffe erzielen lässt. Versuche haben dabei gezeigt, dass die Wellenlänge der von dem jeweiligen UV-Strahler abgegebenen Strahlung zur Erreichung der Katalysatorwirkung mit Titandioxyd vorzugsweise im Bereich zwischen 350 nm und 420 nm liegt.

Die erfindungsgemäße Reaktionsstufe kann dazu verwendet werden, um die aus der EP 0 778 070 B1 bekannte Vorrichtung zur Reinigung schadstoffhaltiger Abluft in einem Abluftkanal hinsichtlich Abbaurate und Abmessungen zu verbessern.

Eine weitere Lösung der vorliegenden Erfindung besteht demnach in einer Vorrichtung zur Reinigung schadstoffhaltiger Abluft in einem Abluftkanal mit der oben beschriebenen erfindungsgemäßen Reaktionsstufe und mit einer dieser Reaktionsstufe nachgeschalteten Katalysatoreinheit.

Mit dieser Vorrichtung gelingt die Bereitstellung einer kostengünstigen Kompaktanlage, die insbesondere für geringe Volumenströme und kleine Produktionseinheiten geeignet ist, wie z. B. kleine Lackierbetriebe oder Restaurants.

Nach einer bevorzugten Ausführungsform besteht die Katalysatoreinheit aus einem Aktivkohle-Katalysator. Wie bereits oben beschrieben, bewirkt die nachgeschaltete Katalysatoreinheit sowohl eine Steigerung der Reaktionsgeschwindigkeit des von der Reaktionsstufe gelieferten Luftstroms als auch den Abbau von Ozon, das in dem ankommenden Luftstrom noch enthalten ist, aber nicht in die Umwelt abgegeben werden soll. Gerät überschüssiges Ozon somit an die Aktivkohle-Oberfläche, so reagiert es entweder mit an der Oberfläche adsorbierten Schadstoffen oder oxidiert den Kohlenstoff der Aktivkohle. Letzteres bedeutet einen Energieverlust, da das mit Hilfe von Lichtenergie erzeugte Ozon ungenutzt, d. h. ohne eine Schadstoffoxidation durchgeführt zu haben, verloren geht.

Nach einer bevorzugten Ausführungsform wird daher vorgeschlagen, ein Redoxsystem bereitzustellen, das zwar den Austritt von Ozon in die Umwelt sicher verhindert, aber dabei die Oxidationskraft des Ozons speichert. Als Redox-Paar bietet sich dabei beispielsweise Kaliumpermangant/Mangandioxyd an. Durch die Oxidation von organischen Schadstoffen durch Kaliumpermangant bildet sich Mangandioxyd, das wiederum durch die Reaktion mit Ozon zum Kaliumpermangant regeneriert wird.

Bei der Bereitstellung der nachgeschalteten Kathalysatoreinheit ist weiterhin zu beachten, dass die in der Praxis abzubauenden Schadstoffgemische im Allgemeinen aus einer Vielzahl unterschiedlicher Substanzen bestehen, da oftmals Schadstoffgemische mit einer Hauptkomponente und mehreren Nebenbestandteilen zu entsorgen sind. Daneben entstehen durch die Photooxidation in der Reaktionsstufe ständig weitere Schadstoffe, die ebenfalls noch in der nachgeschalteten Kathalysatoreinheit abgebaut werden müssen. Da die Oxidationsreaktionen organische Verbindungen nach komplexen Reaktionsmechanismen ablaufen, kann die Oxidation der Schadstoffe zu CO₂ oft erst durch eine Abfolge mehrerer Oxidationsschritte erreicht werden. Im Verlauf der Gesamtreaktion zum Endprodukt CO₂ steigt dabei die Polarität der organischen Verbindungen. Die Komplexität des Schadstoffgemisches führt dabei zu einer Konkurrenz der Komponenten um die Adsorptionsplätze in der Katalysatoreinheit. Dies bedeutet aber, dass ein einziges Adsorbermaterial nicht mehr in der Lage ist, sämtliche Verbindungen eines komplexen Schadstoffgemisches hinreichend zu adsorbieren. So adsorbiert beispielsweise Aktivkohle als unpolarer Adsorber bevorzugt auch unpolare Schadstoffe.

Nach einer weiteren bevorzugten Ausführungsform ist daher vorgesehen, dass die Katalysatoreinheit aus Katalysatoren unterschiedlicher Polarität besteht. Hierdurch kann eine zusätzliche Steigerung der Abbaurate erreicht werden, wenn die Schadstoffe in der von der Reaktionsstufe gelieferten Abluft verschiedene Polaritäten aufweisen.

Nach einer weiteren bevorzugten Ausführungsform ist vorgesehen, dass mehrere Einheiten bestehend aus Reaktionsstufe und nachgeschalteter Katalysatoreinheit hintereinander geschaltet sind. Durch die Bereitstellung mehrerer Katalysatoreinheiten mit jeweils nachfolgenden Reaktionsstufen kann die Auslegung einer Abluftreinigungsanlage bei instationären Schadstoffbelastungen des Rohgases auf die mittlere Schadstoffkonzentration optimiert werden. Bei nur einer Katalysatoreinheit muss die Auslegung hinsichtlich der maximal auftretenden Schadstoffkonzentration erfolgen, was zu großen und damit teuren Anlagen führt. Bei Lackierprozessen liegen aber beispielsweise produktionsbedingt instationäre Schadstoffbelastungen des Abgases vor. Durch den Einsatz zwischengeschalteter Katalysatoreinheiten mit nachfolgenden Reaktionsstufen werden dabei Schadstoffspitzen abgefangen und können nicht "durchbrechen". Trifft eine Schadstoff-Konzentrationsspitze auf eine Katalysatoreinheit, so werden die Schadstoffe adsorbiert und auf der Katalysatoroberfläche umgesetzt bzw. nur langsam wieder an die Gasphase abgegeben, um von einer weiteren nachfolgenden Reaktionsstufe abgebaut werden zu können. Hierdurch kann die Abbaurate des Gesamtsystems weiter erhöht werden und das System auch bei starken Konzentrationsschwankungen verlässlich ausgelegt werden. Somit führt die Hintereinanderschaltung mehrerer Reaktionsstufen und Katalysatoreinheiten letztlich zu einer kompakteren Anlage und damit zu einer Kostensenkung.

Im Folgenden wird die Erfindung anhand verschiedener Ausführungsbeispiele mit Bezug auf die beiliegenden Zeichnungen näher erläutert. Diese zeigen:
- Fig. 1:: den Querschnitt und eine perspektivische Ansicht eines erfindungsgemäßen Luftleitkanals,
- Fig. 2:: eine perspektivische Ansicht einer erfindungsgemäßen Reaktionsstufe mit mehreren parallelen Luftleitkanälen und
- Fig. 3:: eine perspektivische Ansicht einer Abluftreinigungsanlage mit erfindungsgemäßen Reaktionsstufen.

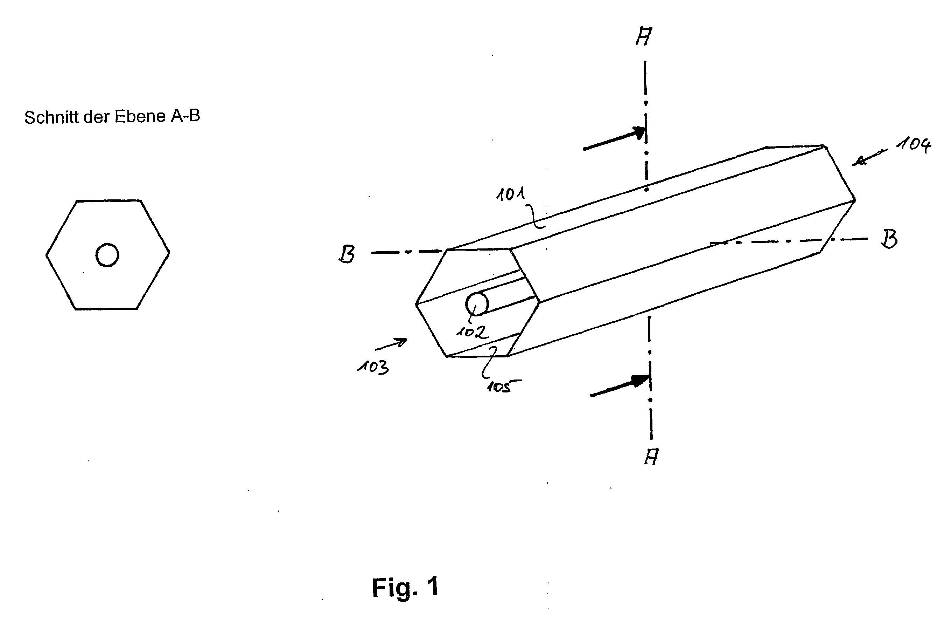

Fig. 1 zeigt den Querschnitt und eine perspektivische Ansicht eines erfindungsgemäßen Luftleitkanals. Wie aus dem Querschnitt der Ebene A-B zu erkennen ist, weist der Luftleitkanal 101 den Querschnitt eines regelmäßigen Sechsecks auf. Mittig im Luftleitkanal 101 ist ein röhrenförmiger UV-Strahler 102 angeordnet. Die schadstoffbelastete Abluft tritt dabei in die Eintrittsöffnung 103 ein und wird aus der Austrittsöffnung 104 wieder abgegeben. Zur Erzielung einer Katalysatorwirkung bereits innerhalb des Luftleitkanals 101 sind die Innenwände 105 mit einem breitbandingen Halbleitermaterial beschichtet, beispielsweise Titandioxyd oder dotiertes Titandioxyd.

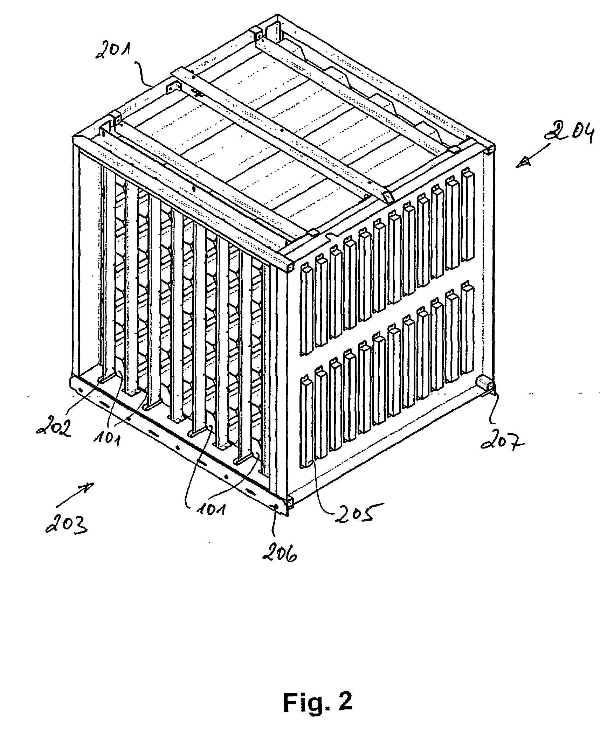

Fig. 2 zeigt eine perspektivische Ansicht einer erfindungsgemäßen Reaktionsstufe mit mehreren parallelen Luftleitkanälen. Die einzelnen Luftleitkanäle 101 entsprechen dabei dem in Fig. 1 dargestellten Luftleitkanal und sind wabenförmig parallel geschaltet. In entsprechender Weise ist in jedem Luftleitkanal 101 jeweils ein röhrenförmiger UV-Strahler angeordnet. Die in dieser Weise zusammen geschalteten Luftleitkanäle 101 sind von einem Metallgehäuse umgeben und bilden damit die Reaktionsstufe 201. An der Lufteintrittsöffnung 203 und der Luftaustrittsöffnung 204 sind jeweils Kontaktschienen 202 vorgesehen, die zum einen als Kabelkanäle für die elektrischen Zuführungen der UV-Strahler dienen und die zum anderen die UV-Strahler mechanisch in den Luftleitkanälen 101 halten. Zur elektrischen Ansteuerung der UV-Strahler sind seitlich entsprechende Vorschaltgeräte 205 vorgesehen. An den Unterseiten der Reaktionsstufe 201 sind Gleitschienen 206 und 207 vorgesehen, damit die Reaktionsstufe 201 im Gesamtsystem zu Wartungszwecken auf entsprechenden Rollen ein- bzw. ausgeschoben werden kann.

Eine weitere Verbesserung der Abbaurate ist zu erreichen, wenn die Innenwandungen des Luftleitkanals mit einem Katalysatormaterial beschichtet werden. Durch die wabenförmige Konstruktion der Reaktionsstufe mit mehreren Luftleitkanälen können große Katalysatoroberflächen in direkter Nähe der UV-Strahlung mit geringem Druckverlust zur Verfügung gestellt werden. Durch die direkte Bestrahlung der Katalysatoroberfläche ergibt sich die Möglichkeit, breitbandige Halbleitermaterialien auf effektive Weise für eine Photokatalyse zu nutzen. Als Katalysatormaterial hat sich insbesondere Titandioxyd als geeignet herausgestellt. Durch Bestrahlung des Titandioxyds mit UV-Licht, dessen Energie größer oder gleich der Energiedifferenz zwischen Valenz- und Leitungsband des Halbleiters ist, werden zunächst Elektronen/Loch-Paare im Halbleitermaterial generiert. Daraufhin kommt es zur Bildung reaktiver O₂⁻-Spezies, die den Prozess der Oxidation von Schadstoffen wirkungsvoll unterstützen. Zur Einleitung dieses Prozesses werden UV-Strahler mit Wellenlängen im Bereich zwischen 340 nm und 420 nm verwendet.

Anschließend erfolgt eine Adsorption von Gasmolekülen an den generierten Ladungen durch Lichteinstrahlung erzeugten Elektron/Loch-Paare. Die nun koadsorbierten Moleküle sind aktiviert und bilden einen Übergangszustand, aus dem heraus sie unter Bildung von Zwischenprodukten zu den Endprodukten reagieren. Die ungefährlichen Reaktionsprodukte desorbieren und können an die Umgebung abgegeben werden. Die photokatalytische Umsetzung läßt sich demnach in vier Schritte einteilen:
1. Erzeugung der Ladungspaare
2. Adsorption der Gase an den generierten Ladungen
3. Reaktion zwischen benachbart adsorbierten reaktiven Molekülen
4. Desorption der Produkte

Mit Hilfe der heterogenen Photokatalyse ist es beispielsweise möglich, Verbindungen wie Ammoniak, Formaldehyd oder niedere Alkohole, die mit der Photooxidation nur schwer zu oxidieren sind, mit hoher Effizienz bei Raumtemperatur mit Luftsauerstoff zu Stickstoff bzw. CO₂ und Wasser zu verbrennen. Der bereits allgemein beschriebene Reaktionsablauf ist in diesem Fall wie folgt:

Die Abluft wird in einen Reaktionskanal geleitet, in dem sich durch UV-Licht aktiviertes Titandioxid befindet. Die Bestrahlung des Photohalbleiters führt zur Generierung von Elektron/Loch-Paaren. Anschließend erfolgt eine Adsorption von Gasmolekülen an den generierten Ladungen, wobei der Energiegewinn bei der Adsorption entscheidet, welche Moleküle bevorzugt mit den Elektronen und welche mit den Löchern wechselwirken. Bei den Reaktionspartnern Ammoniak und Sauerstoff reagiert aufgrund der jeweiligen Moleküleigenschaften Ammoniak mit den Löchern und Sauerstoff mit den Elektronen. Die nun koadsorbierten Moleküle sind aktiviert und bilden einen Übergangszustand, aus dem heraus sie unter Bildung von Zwischenprodukten zu den Endprodukten reagieren. Die ungefährlichen Reaktionsprodukte Stickstoff und Wasser desorbieren und können an die Umgebung abgegeben werden.

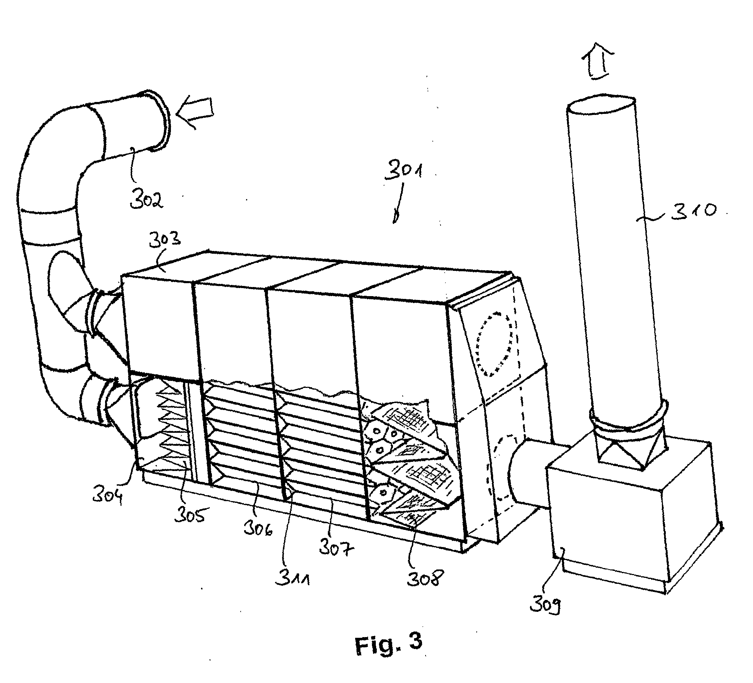

Fig. 3 zeigt eine perspektivische Ansicht einer Abluftreinigungsanlage 301 mit erfindungsgemäßen Reaktionsstufen 306 und 307. Die Reaktionsstufen 306 und 307 entsprechen dabei jeweils der in Fig. 2 dargestellten Reaktionsstufe 201. Die schadstoffhaltige Abluft wird der Abluftreinigungsanlage 301 über ein Zuführrohr 302 zugeführt. Optional können zur Steigerung der zu reinigenden Luftmengen zwei baugleiche Systeme 303 und 304 vorgesehen sein, die in der Darstellung gemäß Fig. 3 übereinander angeordnet sind. Der Einfachheit halber wird im Folgenden nur das System 304 beschrieben, dessen einzelne Komponenten mit Hilfe eines Durchbruchs näher dargestellt sind.

Demnach schließt sich dem Zuführrohr 302 zunächst eine Verteilerstufe 305 an, die die ankommende Luft gleichmäßig verteilt und gegebenenfalls größere Schadstoffpartikel herausfiltert. Die von der Verteilerstufe 305 weitergegebene Luft gelangt in die erfindungsgemäßen Reaktionsstufen 306 und 307. Zur Erhöhung der Abbaurate sind hierbei zwei baugleiche Reaktionsstufen 306 und 307 hintereinander geschaltet. Selbstverständlich kann die Abluftreinigungsanlage 301 aber auch nur mit einer Reaktionsstufe 306 aufgebaut werden. Den beiden Reaktionsstufen 306 und 307 schließt sich eine Katalysatoreinheit 308 an, die in der oben beschriebenen Weise beispielsweise aus geschüttetem hochporösen Aktivkohle-Material mit einer inneren Oberfläche von ca. 1200 m²/g bestehen kann, die als Reaktionsoberfläche genutzt werden.

Die von der Katalysatoreinheit 308 abgegebene Luft gelangt weiter in die Gebläseeinheit 309, die für die Aufrechterhaltung eines entsprechenden Druckunterschiedes zwischen dem Zuführrohr 302 und dem Abführrohr 310 sorgt.

Die Abluftreinigungsanlage 301 wird grundsätzlich nach dem Verfahren gemäß der EP 0 778 070 B1 betrieben, zeichnet sich allerdings erfindungsgemäß durch eine oder mehrere Reaktionsstufen 306, 307 aus, wie diese gemäß Fig. 2 dargestellt ist. Die schadstoffbelastete Abluft gelangt demnach von dem Zuführrohr 302 über die Verteilerstufe 304 in die Reaktionsstufen 306 und 307, in denen kurzwelliges UVC Licht eine chemische Reaktion einleitet. Geruchs- und Schadstoffmoleküle werden dabei aufgebrochen. Gleichzeitig werden Schadstoffradikale und Ozon als Oxidationsmittel erzeugt. Die Oxidation der Schadstoffe führt zu den umweltverträglichen Produkten CO₂ und H₂O. In der nachgeschalteten Katalysatoreinheit 308 werden schwer oxidierbare Verbindungen und überschüssiges Ozon abgebaut. Die gereinigte und geruchslose Luft wird über die Gebläseeinheit 309 und das Abführrohr 310 an die Umwelt abgegeben.

Zur effektiven Behandlung instationärer Schadstoffbelastungen kann an der Stelle 311 in der oben beschriebenen Weise eine weitere Katalysatoreinheit zwischengeschaltet werden. Durch die weitere zwischengeschaltete Katalysatoreinheit gelingt es, auch kurzzeitig auftretende Schadstoffe mit sehr hohen Konzentrationen abzubauen.

## Patentansprüche

1. Reaktionsstufe eines Abluftkanals mit mehreren Luftleitkanälen, wobei die Luftleitkanäle wabenförmig nebeneinander angeordnet sind, **dadurch gekennzeichnet, dass** in den Luftleitkanälen längs zur Luftströmung der Abluft jeweils ein röhrenförmiger UV-Strahler angeordnet ist,
wobei der Querschnitt eines jeden Luftleitkanals ein regelmäßiges Vieleck mit mindestens 5 Seiten ist.

2. Reaktionsstufe nach Anspruch 1, **dadurch gekennzeichnet, dass** der Querschnitt der Luftleitkanäle jeweils ein regelmäßiges Sechseck ist.

3. Reaktionsstufe nach Anspruch 1, **dadurch gekennzeichnet, dass** der Querschnitt der Luftleitkanäle jeweils ein Kreis ist.

4. Reaktionsstufe nach einem der Ansprüche 1 - 3, **dadurch gekennzeichnet, dass** ein UV-Strahler in einem Luftleitkanal durch seitlich angebrachte Kontaktschienen gehalten wird.

5. Reaktionsstufe nach einem der Ansprüche 1 - 4, **dadurch gekennzeichnet, dass** die von einem UV-Strahler abgegebene Strahlung die Bildung von reaktiven Reaktionsmitteln wie Ozon und/oder sauerstoffhaltige Radikale in der entlang strömenden Abluft bewirkt.

6. Reaktionsstufe nach Anspruch 5, **dadurch gekennzeichnet, dass** die Wellenlänge der von dem jeweiligen UV-Strahler abgegebenen Strahlung im Bereich von 185 nm liegt.

7. Reaktionsstufe nach einem der Ansprüche 1 - 6, **dadurch gekennzeichnet, dass** die von einem UV-Strahler abgegebene Strahlung die Anregung der in der Abluft enthaltenen Kohlenwasserstoffe auf höhere energetische Niveaus bewirkt.

8. Reaktionsstufe nach Anspruch 7, **dadurch gekennzeichnet, dass** die Wellenlänge der von dem jeweiligen UV-Strahler abgegebenen Strahlung im Bereich von 254 nm liegt.

9. Reaktionsstufe nach einem der Ansprüche 1 - 8, **dadurch gekennzeichnet, dass** die Innenwände der Luftleitkanäle mit einem breitbandigen Halbleitermaterial als Katalysatormaterial beschichtet sind.

10. Reaktionsstufe nach Anspruch 9, **dadurch gekennzeichnet, dass** die von dem jeweiligen UV-Strahler abgegebene Strahlung Wellenlängen aufweist, die größer als 254 nm sind und deren abgegebene Strahlungsenergie im Wesentlichen größer oder gleich der Energiedifferenz zwischen Valenz- und Leitungsband des Halbleitermaterials ist.

11. Reaktionsstufe nach einem der Ansprüche 9 - 10, **dadurch gekennzeichnet, dass** die von dem jeweiligen UV-Strahler abgegebene Strahlung Wellenlängen aufweist, die im Bereich der Absorptionskante des Halbleitermaterials liegen.

12. Reaktionsstufe nach einem der Ansprüche 9 - 11, **dadurch gekennzeichnet, dass** die von dem jeweiligen UV-Strahler abgegebene Strahlung Wellenlängen aufweist, die im Bereich zwischen 340 nm und 500 nm, vorzugsweise zwischen 350 nm und 420 nm, liegen.

13. Reaktionsstufe nach einem der Ansprüche 9 - 12, **dadurch gekennzeichnet, dass** das Halbleitermaterial aus Titandioxid (TiO₂) oder dotiertem Titandioxid besteht.

14. Reaktionsstufe nach einem der Ansprüche 9 - 12, **dadurch gekennzeichnet, dass** das Halbleitermaterial aus Zinkoxid (ZnO), Cadmiumsulfat (CdS), Zirkoniumdioxid (ZrO₂), Wolframtrioxid (WO₃), Cerdioxid (CeO₂), Strontiumtitamtrioxid (SrTiO₃) oder Zirkoniumtitanoxid (ZrTiO₄) besteht.

15. Vorrichtung zur Reinigung schadstoffhaltiger Abluft in einem Abluftkanal,
mit einer Reaktionsstufe nach einem der Ansprüche 1 - 14 und
mit einer der Reaktionsstufe nachgeschalteten Katalysatoreinheit.

16. Vorrichtung nach Anspruch 15, **dadurch gekennzeichnet, dass** die Katalysatoreinheit aus einem Aktivkohle-Katalysator besteht.

17. Vorrichtung nach Anspruch 15, **dadurch gekennzeichnet, dass** die Katalysatoreinheit auf einem Redoxsystem basiert.

18. Vorrichtung nach Anspruch 17, **dadurch gekennzeichnet, dass** das Redoxsystem durch die Komponenten Kalimpermangant / Mangandioxid gebildet wird.

19. Vorrichtung nach Anspruch 15, **dadurch gekennzeichnet, dass** die Katalysatoreinheit aus Katalysatoren unterschiedlicher Polarität besteht.

## Claims

1. Reaction stage of a used air duct comprising a plurality of air conduits, wherein the air conduits are arranged next to one another in a honeycombed configuration,
**characterized in**
**that** in the air conduits a respective tubular UV emitter is arranged longitudinally to the direction of flow of the used air,
wherein the cross section of each air conduit is configured as a regular polygon having at least five sides.

2. Reaction stage according to claim 1, **characterized in that** the cross section of the air conduits is configured as a respective regular hexagon.

3. Reaction stage according to claim 1, **characterized in that** the cross section of the air conduits is configured as a respective circle.

4. Reaction stage according to any one of claims 1 to 3, **characterized in that** a UV emitter is held in an air conduit by means of laterally attached contact rails.

5. Reaction stage according to any one of claims 1 to 4, **characterized in that** the radiation emitted by a UV emitter causes the formation of reactive reactants such as ozone and/or oxygen-containing radicals in the used air as it flows along.

6. Reaction stage according to claim 5, **characterized in that** the wavelength of the radiation emitted by the respective UV emitter is in the range of 185 nm.

7. Reaction stage according to any one of claims 1 to 6, **characterized in that** the radiation emitted by a UV emitter causes the stimulation of the hydrocarbons contained in the used air to higher energy levels.

8. Reaction stage according to claim 7, **characterized in that** the wavelength of the radiation emitted by the respective UV emitter is in the range of 254 nm.

9. Reaction stage according to any one of claims 1 to 8, **characterized in that** the internal walls of the air conduits are coated with a broadband semiconductor material as a catalyst material.

10. Reaction stage according to claim 9, **characterized in that** the radiation emitted by the respective UV emitter has wavelengths that are greater than 254 nm and the emitted radiation energy of which is substantially greater than or equal to the energy differential between the valence and conduction bands of the semiconductor material.

11. Reaction stage according to either claim 9 or claim 10, **characterized in that** the radiation emitted by the respective UV emitter has wavelengths located in the range of the absorption edge of the semiconductor material.

12. Reaction stage according to any one of claims 9 to 11, **characterized in that** the radiation emitted by the respective UV emitter has wavelengths located in the range between 340 nm and 500 nm, preferably between 350 nm and 420 nm.

13. Reaction stage according to any one of claims 9 to 12, **characterized in that** the semiconductor material consists of titanium dioxide (TiO₂) or doped titanium dioxide.

14. Reaction stage according to any one of claims 9 to 12, **characterized in that** the semiconductor material consists of zinc oxide (ZnO), cadmium sulphate (CdS), zirconium dioxide (ZrO₂), tungsten trioxide (WO₃), cerium dioxide (CeO₂), strontium titanium trioxide (SrTiO₃) or zirconium titanium oxide (ZrTiO₄).

15. Device for purifying used air containing harmful substances in a used air duct, comprising a reaction stage according to any one of claims 1 to 14 and comprising a catalyst unit following the reaction stage.

16. Device according to claim 15, **characterized in that** the catalyst unit consists of an activated carbon catalyst.

17. Device according to claim 15, **characterized in that** the catalyst unit is based on a redox system.

18. Device according to claim 17, **characterized in that** the redox system is formed by the components potassium permanganate/manganese dioxide.

19. Device according to claim 15, **characterized in that** the catalyst unit consists of catalysts of different polarities.

## Revendications

1. Etage de réaction d'un canal d'air d'évacuation comprenant plusieurs canaux de guidage d'air, les canaux de guidage d'air étant disposés les uns à côté des autres en forme de nid d'abeilles, **caractérisé en ce que** dans les canaux de guidage d'air, dans la direction d'écoulement d'air d'évacuation, est chaque fois disposé un émetteur UV à forme tubulaire,
la section transversale de chaque canal de guidage d'air étant un polygone régulier comprenant au moins 5 côtés.

2. Etage de réaction selon la revendication 1, **caractérisé en ce que** la section transversale des canaux de guidage d'air est chaque fois un hexagone régulier.

3. Etage de réaction selon la revendication 1, **caractérisé en ce que** la section transversale des canaux de guidage d'air est chaque fois un cercle.

4. Etage de réaction selon l'une des revendications 1 à 3, **caractérisé en ce qu'**un émetteur d'UV est maintenu dans un canal de guidage d'air par des glissières de contact montées latéralement.

5. Etage de réaction selon l'une des revendications 1 à 4, **caractérisé en ce que** le rayonnement émis par un émetteur d'UV provoque la formation de produits de réaction réactifs, tels que de l'ozone et/ou des radicaux contenant de l'oxygène, dans l'air d'évacuation qui s'écoule le long de l'émetteur.

6. Etage de réaction selon la revendication 5, **caractérisé en ce que** la longueur d'ondes du rayonnement émis par l'un émetteur d'UV respectif est de l'ordre de 185 nm.

7. Etage de réaction selon l'une des revendications 1 à 6, **caractérisé en ce que** le rayonnement émis par un émetteur d'UV provoque l'excitation à un niveau énergétique plus élevé des hydrocarbures contenus dans l'air d'évacuation.

8. Etage de réaction selon la revendication 7, **caractérisé en ce que** la longueur d'ondes du rayonnement émis par l'émetteur d'UV respectif est de l'ordre de 254 nm.

9. Etage de réaction selon l'une des revendications 1 à 8, **caractérisé en ce que** les parois intérieures des canaux de guidage d'air sont revêtues d'un matériau semi-conducteur à bande large, faisant office de matériau catalyseur.

10. Etage de réaction selon la revendication 9, **caractérisé en ce que** le rayonnement émis par l'émetteur d'UV présente des longueurs d'ondes supérieures à 254 nm et dont l'énergie de rayonnement délivrée est sensiblement supérieure ou égale à la différence d'énergie entre la bande de valence et la bande de conductivité du matériau à semi-conducteur

11. Etage de réaction selon l'une des revendications 9 à 10, **caractérisé en ce que** le rayonnement émis par l'émetteur d'UV respectif présente des longueurs d'ondes dans la zone de la limite d'absorption du matériau semi-conducteur.

12. Etage de réaction selon l'une des revendications 9 à 11, **caractérisé en ce que** le rayonnement émis par l'émetteur d'UV respectif présente des longueurs d'ondes dans la fourchette comprise entre 340 nm et 500 nm, de préférence entre 350 nm et 420 nm.

13. Etage de réaction selon l'une des revendications 9 à 12, **caractérisé en ce que** le matériau semi-conducteur est composé de dioxyde de titane (TiO₂) ou de dioxyde de titane dopé.

14. Etage de réaction selon l'une des revendications 9 à 12, **caractérisé en ce que** le matériau semi-conducteur est composé d'oxyde de zinc (ZnO), de sulfate de cadmium (CdS), de dioxyde de zirconium (ZrO₂), de trioxyde de tungstène (WO₃), de dioxyde de cérium (CeO₂), de trioxyde de strontium-titane (SrTiO₃) ou d'oxyde de zirconium-titane (ZrTiO₄).

15. Dispositif d'épuration de l'air d'évacuation contenant des substances nuisibles, dans un canal d'évacuation d'air,
avec un étage de réaction selon l'une des revendications 1 à 14, et
avec une unité de catalyseur installée en aval de l'étage de réaction.

16. Dispositif selon la revendication 15, **caractérisé en ce que** l'unité de catalyseur est composée d'un catalyseur à charbon actif.

17. Dispositif selon la revendication 15, **caractérisé en ce que** l'unité de catalyseur est basée sur un système redox.

18. Dispositif selon la revendication 17, **caractérisé en ce que** le système redox est formé par les composants permanganate de potassium/dioxyde de manganèse.

19. Dispositif selon la revendication 15, **caractérisé en ce que** l'unité de catalyseur est composée de catalyseurs à polarité différente.
